(19) Europäisches Patentamt
European Patent Office
Office européen des brevets

(11) Publication number: **0 057 536 A1**

# (12) EUROPEAN PATENT APPLICATION

(21) Application number: **82300323.1**

(22) Date of filing: **22.01.82**

(51) Int. Cl.³: **C 07 D 513/04**, C 07 D 498/04, A 61 K 31/535, A 61 K 31/54 // (C07D513/04, 279/00, 221/00), (C07D513/04, 279/00, 209/00), (C07D498/04, 265/00, 221/00)

(30) Priority: **26.01.81 GB 8102319**

(43) Date of publication of application: **11.08.82 Bulletin 82/32**

(84) Designated Contracting States: **BE CH DE FR GB IT LI NL**

(71) Applicant: **BEECHAM GROUP PLC, Beecham House Great West Road, Brentford Middlesex (GB)**

(72) Inventor: **King, Francis David, 67 Cherry garden Lane, Newport Essex (GB)**
Inventor: **Hadley, Michael Stewart, 9 Coney Gree, Sawbridgeworth Hertfordshire (GB)**

(74) Representative: **Stott, Michael John et al, European Patent Attorney Beecham Pharmaceuticals Yew Tree Bottom Road, Epsom Surrey KT18 5XQ (GB)**

(54) **Pharmaceutically active compounds.**

(57) Compounds of the formula (I):

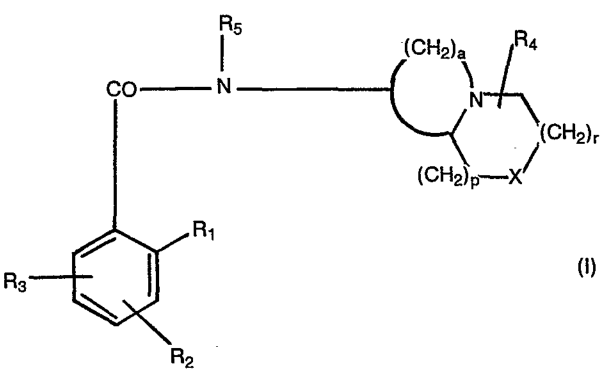

(I)

and pharmaceutically acceptable salts thereof, wherein:

a is 3 to 5; and the $R_5$ substituted nitrogen atom is bound to one of the $-(CH_2)_a-$ carbon atoms, and has an ethan-1,2-diyl moiety linking it and the cyclic nitrogen atom;

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl-$S(O)_n$ wherein n is 0, 1 or 2, nitro, $C_{1-6}$ alkoxy, hydroxy, or amino, aminocarbonyl or aminosulphonyl optionally substituted by one or two $C_{1-6}$ alkyl groups;

or $R_1$ and $R_2$ taken together are methylenedioxy or ethylenedioxy in which case $R_3$ is any one of the groups given for $R_1$ and $R_2$ above;

$R_4$ is hydrogen, $C_{1-4}$ alkyl, phenyl or phenyl-$C_{1-4}$ alkyl;

$R_5$ is hydrogen or $C_{1-4}$ alkyl;

X is an oxygen or sulphur atom; or a sulphoxide group $>S{\sim}O$;

p is 1 or 2; and

r is 1 or 2 having useful pharmaceutical activity, compositions containing them and a process for their preparation.

EP 0 057 536 A1

## PHARMACEUTICALLY ACTIVE COMPOUNDS

This invention relates to a group of novel compounds, their formulation as pharmaceutical compositions, and their use in the therapy of disorders.

West German Offenlegungsschrift No: 2,748,260.6 describes compounds of the formula (A), and pharmaceutically acceptable salts thereof:

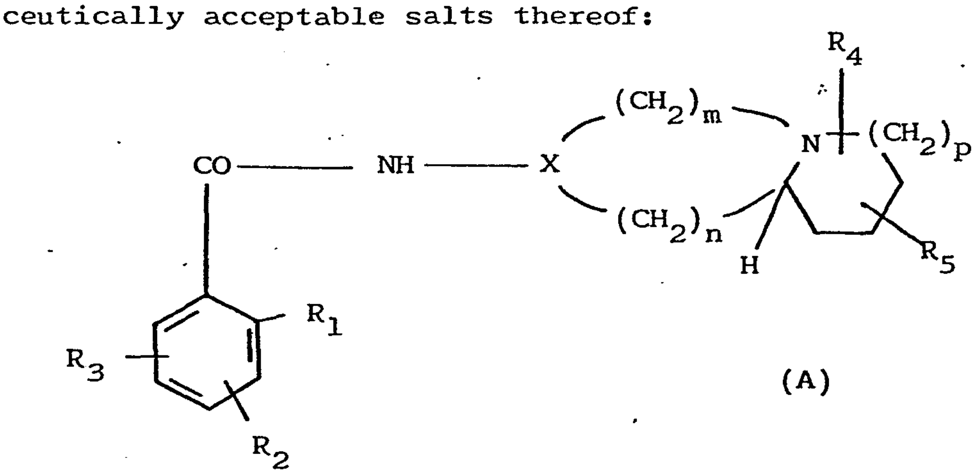

(A)

wherein:

$R_1$ is a $C_{1-6}$ alkoxy group;

$R_2$ and $R_3$ are the same or different and are hydrogen, halogen, $CF_3$, hydroxy, $C_{1-6}$ alkoxy, $C_{2-7}$ acyl, amino, amino substituted by one or two $C_{1-6}$ alkyl groups, $C_{2-7}$ acyl amino, aminocarbonyl or aminosulphone optionally substituted by one or two $C_{1-6}$ alkyl groups, $C_{1-6}$ alkylsulphone or nitro groups;

X is either a nitrogen atom, in which case m+n is 3 to 5, m is 2 to 4 and n is 1 to 3; or X is CH in which case m+n is 2 to 5, m is 1 to 5, and n is 0 to 4; p is 0 to 3; and

$R_4$ is hydrogen, $C_{1-6}$ alkyl, phenyl or phenyl-$C_{1-6}$ alkyl, either of which phenyl moiety may be substituted by $C_{1-6}$ alkyl, $C_{1-6}$ alkoxy, $CF_3$ or halogen, and $R_5$ is hydrogen; or $R_4$ and $R_5$ are attached to two adjacent carbon atoms and form together with these two carbon atoms a fused benzene ring, which benzene ring may be substituted by $C_{1-6}$ alkyl, $C_{1-6}$ alkoxy, $CF_3$ or halogen; as dopamine antagonists having use in the treatment of disorders of the gastro-intestinal function and/or in the treatment of emesis.

A novel class of compounds has now been discovered which is structurally distinct from the known compounds of the formula (I) and also has useful pharmacological activity, such as dopamine antagonist activity.

Accordingly the present invention provides a compound of the formula (I):

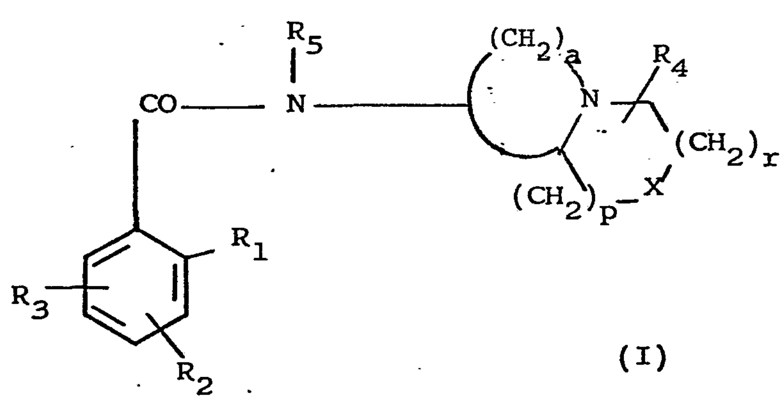

(I)

and pharmaceutically acceptable salts thereof, wherein:

a is 3 to 5; and the $R_5$ substituted nitrogen atom is bound to one of the $-(CH_2)_a-$ carbon atoms, and has an ethan-1,2 - diyl moiety linking it and the cyclic nitrogen atom;

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl-$S(O)_n$ wherein n is O, 1 or 2, nitro, $C_{1-6}$ alkoxy, hydroxy, or amino, aminocarbonyl or aminosulphonyl optionally substituted by one or two $C_{1-6}$ alkyl groups;

or $R_1$ and $R_2$ taken together are methylenedioxy or ethylenedioxy in which case $R_3$ is any one of the groups given for $R_1$ and $R_2$ above;

$R_4$ is hydrogen, $C_{1-4}$ alkyl, phenyl or phenyl-$C_{1-4}$ alkyl;

$R_5$ is hydrogen or $C_{1-4}$ alkyl;

X is an oxygen or sulphur atom; or a sulphoxide group >S~O;

p is 1 or 2; and

r is 1 or 2.

- - -

Suitable examples of a are 3 and 4.

There must be an ethan - 1,2 - diyl moiety linking the two nitrogen atoms in formula (I). Thus the relevant portion of formula (I) can be represented by the partial structures $(I)_a$ and $(I)_b$:

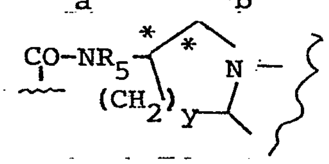

$(I)_a$

wherein y is 1 to 3;

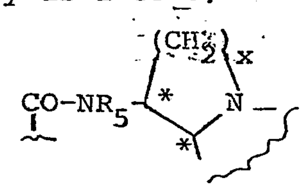

$(I)_b$

wherein x is 2 to 4.

The ethan - 1,2 - diyl moiety is formed by the two carbon atoms marked *

Suitable examples of the group $R_1$ include methoxy, ethoxy and *n*- and *iso*-propoxy. Preferably $R_1$ is a methoxy group.

Suitable examples of $R_2$ and $R_3$ include the following atoms and groups: hydrogen; chlorine, bromine; $CF_3$; formyl, acetyl, propionyl, n- and iso-butyryl; formylamino, acetylamino, propionylamino, n- and iso-butyrylamino; methyl, ethyl and n- and iso-propylsulphone, -sulphinyl or -thia; nitro; methoxy, ethoxy and n- and iso-propoxy; hydroxy; amino, aminocarbonyl and aminosulphonyl and amino, aminocarbonyl, and aminosulphonyl substituted by one or two methyl, ethyl, n- or iso-propyl groups.

When $R_1$ and $R_2$ taken together are methylenedioxy or ethylenedioxy, they are most suitably ethylenedioxy.

Particuarly suitable $R_2$ and $R_3$ groups include hydrogen, halogen and amino; and acylamino and nitro, which can also conveniently be converted to the corresponding amino groups.

It is generally preferred that $R_2$ is in the 4-position relative to the carbonyl side chain, for greater activity in the resultant compound of the formula (I). For the same reason, it is generally preferred that $R_3$ is in the 5-position relative to the carbonyl side chain.

Particularly preferred $R_2$ groups include 4-amino and 4-acylamino. Most preferably $R_2$ is 4-amino. Particularly preferred $R_3$ groups include 5-halo, such as 5-chloro.

In other useful compounds $R_2$ is hydrogen, 4-halo (eg chloro), or amino; and $R_3$ is 5-$C_{1-6}$ alkyl $S(O)_n$ (such as 5-methylsulphonyl, -sulphinyl or -thio) or 5-optionally alkylated aminosulphonyl.

$R_4$ may substitute any carbon in the X-containing ring (apart from the bridgehead carbon), but preferably substitutes the carbon atom adjacent the nitrogen atom.

Suitable examples of $R_4$ include hydrogen, methyl, ethyl, n- and iso-propyl, phenyl and benzyl. Phenyl groups in $R_4$ may be substitued by one or more $C_{1-4}$ alkoxy (such as ethoxy), $C_{1-4}$ alkyl (such as methyl), fluoro, chloro or $CF_3$. Often $R_4$ will be hydrogen, $C_{1-4}$ alkyl or phenyl. Most suitably $R_4$ is hydrogen or methyl.

Suitable examples of $R_5$ include hydrogen, methyl, ethyl and n- and iso-propyl. More suitably $R_5$ is hydrogen or methyl, preferably hydrogen.

Generally X is most suitably oxygen.

(The X is $>S\rightarrow O$ group has been referred to above as the sulphoxide group. The skilled chemist will appreciate that in naming a compound containing such a $>S\rightarrow O$ group, the group can be referred to as "oxothia", and indeed this terminology has been used in some Examples.)

Preferably p is 1.

Preferably r is 1.

It will be appreciated that the bond between the $R_5$ substituted nitrogen atom and the ring carbon atom to which this nitrogen atom is joined in the compounds of formula (I) may be equatorial or axial. (Alternatively, the hydrogen at the ring junction with trans fusion is defined as equatorial α, and the junction of the $R_5$ substituted nitrogen atom is defined as α if on the same side or β if on the opposite side of the plane of the azabicyclic system to this hydrogen.)

The pharmaceutically acceptable salts of the compound of the formula (I) include acid addition salts with conventional acids such as hydrochloric, hydrobromic, phosphoric, sulphuric, citric, tartaric, lactic and acetic acids.

The pharmaceutically acceptable salts of the compounds of the formula (I) also include quaternary ammonium salts. Examples of such salts include salts with compounds such as $R_6$ - Y wherein $R_6$ is $C_{1-6}$ alkyl, phenyl - $C_{1-6}$ alkyl or $C_{5-7}$ cycloalkyl, and Y is an anion of an acid. Suitable examples of $R_6$ include methyl, ethyl and n- and iso-propyl; and benzyl and phenyl ethyl. Suitable examples of Y include the halides such as chloride, bromine and iodide.

Suitable pharmaceutically acceptable salts also include internal salts such as N-oxides.

From the aforesaid it will be seen that suitably the moiety of formula (11):

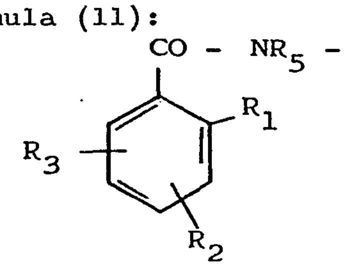

(II)

in a compound of the formula (I) will have the structure (III):

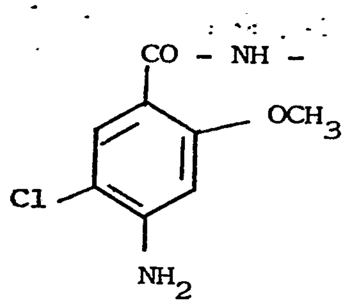

(III)

From the aforesaid it will also be appreciated that preferably the moiety of formula (IV):

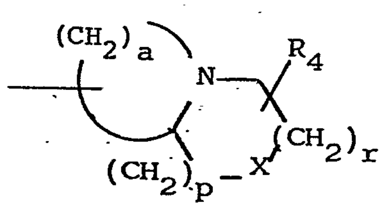

(IV)

in the compound of formula (I) is of the formula (V):

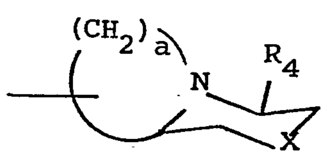

(V)

Thus a preferred sub-group of compounds within formula (I) is of formula (VI):

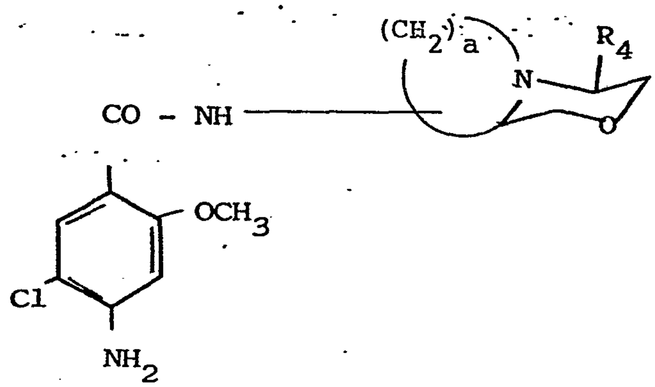

(VI)

In formula (VI) suitably $R_4$ may be hydrogen or methyl.

Another preferred sub-group of compounds within formula (I) is of formula (VII):

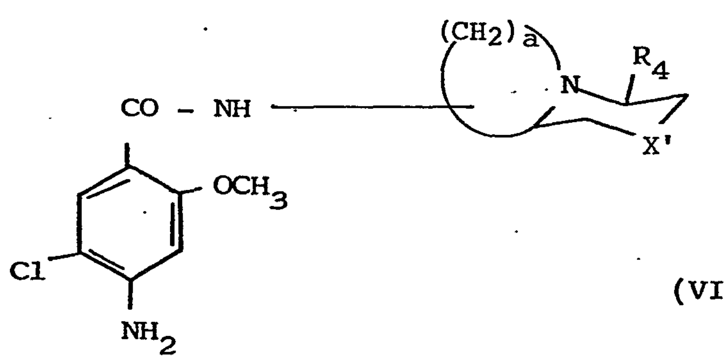

(VII)

wherein X' is S or S→O, preferably S.

Also in formula (VII) suitably $R_4$ may be hydrogen or methyl.

In the formulae (VI) and (VII) a is most suitably 3 or 4.

It will of course be realised that the compounds of the formula (1) have chiral centres, and thus are capable of existing in a number of steroisomeric forms. The invention extends to each of these stereoisomeric forms, including enantiomers, and to mixtures thereof (including racemates). The different steroisomeric forms may be separated one from the other by the usual methods such as treatment with a chiral acid followed by chromatographic or fractional crystallisation separation, or any given isomer may be obtained by stereospecific or asymmetric synthesis.

The invention also provides a process for the preparation of a compound of the formula (I), which process compriese reacting an acid of the formula (VIII):

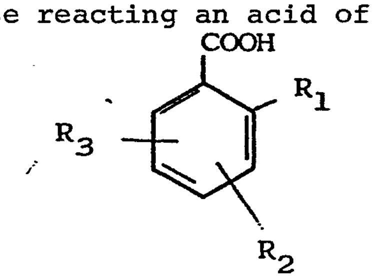

(VIII)

or a reactive derivative thereof, with a compound of the formula (IX):

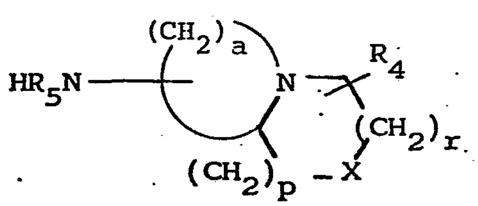

(IX)

wherein the variable groups are as defined in formula (I); and thereafter if desired or necessary converting a group $R_2$, $R_3$ or X in the thus formed compound to another group $R_2$, $R_3$ or X respectively.

"Reactive derivative" when used herein means a derivative of the compound (VIII) which can be reacted with the compound (IX) to form an amido linkage between the acid group of the compound (VIII) and the amino group of the compound of the formula (IX).

Often this reactive derivative will be the acid halide, such as the acid chloride, of the acid (VIII). In such cases the reaction will normally be carried out in an inert solvent, preferably in the presence of an acid acceptor. The inert solvent can be any solvent inert to both reactants, such as benzene, toluene, diethyl ether or the like. The acid acceptor is suitably an organic base such as a tertiary amine, e.g. triethylamine, trimethylamine, pyridine or picoline, or an inorganic acid acceptor, such as calcium carbonate, sodium carbonate, potassium carbonate or the like. It should be noted also that it is possible to use certain acid acceptors as the inert solvent, for example organic bases.

Another useful reactive derivative of the acid (VIII) that may be used is an ester, such as a methyl, ethyl, propyl or butyl ester, in which case the reaction is normally carried out by heating the reactants together in an inert solvent such as ethylene glycol.

The reaction may also be carried out by forming an anhydride of the acid (VIII) in the usual manner, and reacting that with the compound (IX); normally a conventional mixed anhydride will be used; or by reacting the acid (VIII) and the compound (IX) in the presence of a dehydrating catalyst such as a carbodiimide, for example dicyclohexylcarbodiimide.

As stated previously in the compound of the formula (I) the linkage between the $-NR_5-$ moiety and the cyclic side chain may be equatorial or axial.

A mixture of equatorial and axial isomers of the compound of the formula (I) may be synthesised non-stereospecifically and the desired isomer separated conventionally therefrom, e.g. by chromatography; or alternatively the equatorial or axial isomer may if desired be synthesised from the corresponding form of the compound of the formula (IX).

A mixture of the equatorial and axial isomers of the compound of the formula (IX) may be synthesised non-stereospecifically and the desired isomer separated conventionally therefrom, e.g. by chromatography. However, in this case it is generally more convenient to react the mixture to give a mixture of equatorial and axial isomers of the compound of the formula (I) and to separate these if desired as hereinbefore described.

Alternatively, a given isomer of the compound of the formula (IX) may be synthesised stereospecifically.

The compounds of formula (IX) may be prepared by a process which depends on whether the equatorial or axial form is desired, or a mixture thereof.

To prepare equatorial compounds of formula (IX), a compound of formula (X) is reduced:

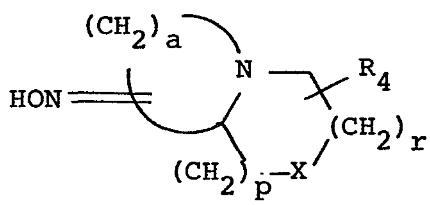

(X)

This reduction is suitably carried out by a dissolving metal reduction, for example with sodium and amyl alcohol. Any minor proportion of the undesired axial isomer also produced as a side product may be separated in conventional manner, for example by chromatography.

Alternatively, reduction of the compound of formula (X) with lithium aluminium hydride gives a mixture of equatorial and axial isomers, from which mixture if so desired the axial isomer can be separated conventionally.

Compounds of formula (X) may themselves be prepared from a compound of formula (XI) by reaction with hydroxylamine:

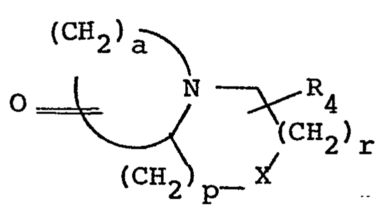

(XI)

This reaction is suitably carried out with pyridine and hydroxylamine hydrochloride at reflux temperature.

The compounds of formula (XI) may be prepared by the Dieckmann cyclisation of a compound of formula (XII):

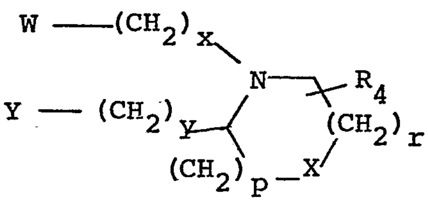

(XII)

wherein x and y are as defined in formula $(I)_a$ and $(I)_b$ above, but in formula (XII) there is the necessary proviso that when x is 2 to 4, y is 0, and when y is 1 to 3, x is 1; and W and Y are both esters, such as $C_{1-4}$ alkyl esters (for example ethyl esters), or one or both of W and Y are nitriles.

Suitable conditions for this reaction are standard, for example potassium tert-butoxide in diethyl ether at room temperature followed by acid hydrolysis with a dilute mineral acid, such as hydrochloric acid.

Compounds of the formula (XII) may be prepared by the alkylation of a compound of formula (XIII):

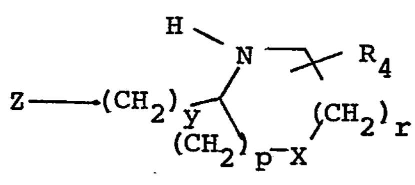

(XIII)

wherein Z is an ester, hydroxy or nitrile function; with a suitably substituted alkyl halide. After this reaction a Z hydroxy can be converted into Y (in formula (XIV)) by conventional methods, for example by reaction with thionyl chloride followed by substitution with a metal cyanide, normally sodium cyanide.

The compounds of formula (XIII) are either known compounds or may be prepared from known compounds in conventional manner.

The aforesaid processes of course yield a compound of the formula (IX) wherein $R_5$ is hydrogen.

If a compound of formula (IX) wherein $R_5$ is alkyl is desired, it may simply be prepared from the corresponding $R_5$ is hydrogen compound by alkylation. This may be effected in any suitable manner, for example by acylation with an anhydride followed by reduction with lithium aluminium hydride.

The compounds of formulae (IX) to (XII) are believed to be novel compounds, and as intermediates form an important part of this invention.

The acid addition salts of compounds of the formula (1) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_6Y$ as defined. This reaction is suitably carried out in an appropriate solvent such as acetone, methanol, ethanol or dimethylformamide at ambient or raised temperature and pressure.

The N-oxides of the compounds of formula (I) may be prepared in conventional manner, for example by treatment with a per-acid, such as m-chloroperbenzoic acid.

The interconversion of suitable groups $R_2$ and $R_3$ after formation of a compound of the formula (I) or corresponding intermediate therefor may be carried out by conventional methods. By way of example nitro groups may be reduced to amino groups in the normal manner, and acylamino groups may be converted to amino groups also by conventional methods. Also a compound of the formula (I) wherein $R_2$ or $R_3$ is halogen can be prepared by a conventional halogenation of the corresponding compound of the formula (I) wherein the said $R_2$ or $R_3$ is hydrogen. Further, compounds wherein $R_2$ or $R_3$ are $C_{1-6}$ alkylsulphonyl may be prepared by oxidation of the corresponding $C_{1-6}$ alkyl sulphinyl compounds; and such $C_{1-6}$ alkylsulphinyl compounds may be prepared by oxidation of the corresponding $C_{1-6}$ alkylthio compound. (it will be appreciated that when X is S it may be preferable to have carried out this step on theacid (VIII) prior to coupling. In regard to X, X is S compounds may be oxidised to X is S→O compounds, suitably

with sodium periodate.

It will be appreciated by the skilled man that, depending on the other specific substituents in the compound of the formula (I), it may not be possible to effect such oxidations on a compound of the formula (I) selectively. In some cases, oxidation may also form the N-oxide of the bicyclic moiety.

Given the specific substitution and X value desired and having decided whether the compound or its N-oxide is required, the skilled man will readily ascertain whether such interconversion is desirable.

In general however it is more convenient to prepare such compounds of formula (I) from the corresponding intermediates, avoiding later interconversion.

Accordingly it will be realised that compounds of the formula (I) containing a $R_2$, $R_3$ or X group which is convertible to another $R_2$, $R_3$ or X group are useful intermediates, and as such form an important aspect of the invention.

As hereinbefore stated, the compounds of the formula (I) are dopamine antagonists.

The compounds of the formula (I) may be used in the treatment of disorders related to impaired gastro-intestinal motility, such as retarded gastric emptying, dyspepsia, flatulence, oesophagal reflux, peptic ulcer and emesis.

The invention therefore also provides a pharmaceutical composition comprising a compound of the formula (I), or a pharmaceutically acceptable salt thereof, together with a pharmaceutically acceptable carrier.

Such compositions may be adapted for oral or parental administration, and as such may be in the form of tablets, capsules, oral liquid preparations, powders, granules, lozenges, reconsitutable powders, injectable and infusable solutions or suspensions and the like; the compositions may also be in the form of suppositories and the like. Normally, orally administrable compositions are preferred.

Tablets and capsules for oral administration may be in unit dose presentation form, and may contain conventional excipients such as binding agents, fillers, tabletting lubricants, disintegrants, and acceptable wetting agents and the like. The tablets may be coated according to methods well known in normal pharmaceutical practice. Oral liquid preparations may be in the form of, for example, aqueous or oily suspensions, solutions, emulsions, syrups, or elixirs, or may be presented in a dry product for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents, and the like.

For parenteral administration, fluid unit dosage forms are prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to

facilitate uniform distribution of the compound.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention further provides a method of treatment of maladies in mammals including humans comprising the administration of an effective amount of a compound of the formula (I) or a pharmaceutically acceptable salt thereof. The "effective amount" will depend in the usual way on a number of factors such as the nature and severity of the malady to be treated, the weight of the sufferer, and the actual compound used.

However by way of illustration, unit doses will suitably contain 0.1 to 20 mgs of the compound of formula (I), for example 0.5 to 10 mgs.

Again by way of illustration, such unit doses will suitably be administered more than once a day, for example 2, 3, 4, 5 or 6 times a day, in such a way that the total daily dose is suitably in the range 0.01 to 10 mg/kg per day.

Compounds of the formula (I) have the ability to potentiate the effect of conventional analgesics in migraine treatment when administered concurrently with the analgesic.

Thus the invention provides a pharmaceutical composition comprising a compound of the formula (I) and an analgesic.

The compound of the formula (I) and the analgesic, such as aspirin or paracetamol, will be present in the composition in amounts generally similar to their usual effective dose.

The composition can be a combination product, for example a tablet or capsule containing both a compound of the formula (I) and an analgesic for oral administration, or a twin pack comprising the two active ingredients made up for separate administration.

The invention accordingly provides a method of treatment of migraine comprising the administration to the sufferer of a compound of the formula (I) and an analgesic.

The following Examples illustrate the preparation of the compounds of formula (I) and the following Descriptions illustrate the preparation of intermediates thereto.

In the Examples, ($\pm$) of course indicates that the compound is a racemate. However, for the sake of convenience, the structural formula of only one enantiomer has been shown.

Description 1

(a) (±) Ethyl 4-(3'-ethoxycarbonyl)morphilino butyrate (D1) intermediate for Compounds (1) and (2)

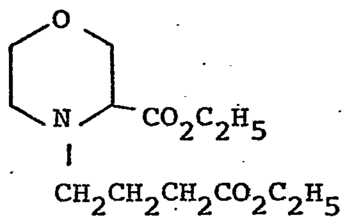

(D1)

A mixture of ethyl 3-morpholino carboxylate (7.5 g), potassium carbonate (9 g) and ethyl 4-bromobutyrate (9.8 g) was heated on a steam bath for 3 hours. On cooling, ether (200 ml) and water (50 ml) were added and the organic layer was separated. The product was extracted into 2N hydrochloric acid (100 ml), neutralised with potassium carbonate and re-extracted into ether (2 x 100 ml). Removal of the solvent in vacuo gave the crude (±) ethyl 4-(3'-ethoxycarbonyl)morpholino butyrate (D1) (9 g, 70%).

Mass Spectrum: ($M^+$) Found, 273.1577, theory 273.1566.

(±) Ethyl 4- (3'ethoxycarbonyl) thiomorpholino butyrate (D5) intermediate for compounds (3) and (4)

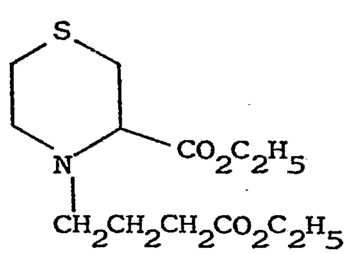

(D5)

Analogously, ethyl 3-thiomorpholine carboxylate (17 g) was converted to (±) ethyl 4- (3'-ethoxycarbonyl) thiomorpholino butyrate (D5) (22 g) 75%)

Mass Spectrum, ($M^{\oplus}$) 289

(b) (±) 1-Aza-6α-H-4-oxabicyclo[4,4,0]decane-7-one (D2) intermediate for compounds (1) and (2)

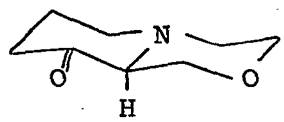

(D2)

To a stirred suspension of potassium t-butoxide (12 g) in ether (200 ml), at room temperature, was added the (±) ethyl 4-(3'-ethoxycarbonyl)morpholino butyrate (D1) (9 g) in ether (100 ml) over 1 hour and the mixture was stirred for a further 2 hours. The reaction mixture was extracted with 5N hydrochloric acid (200 ml) and the acid extract was heated at reflux for 3 hours. Evaporation to dryness, addition of saturated potassium carbonate solution and extraction with methylene chloride gave, after evaporation of the solvent, the crude (±) 1-aza-6α-H-4-oxabicyclo[4,4,0]decane-7-one (D2) (4.0 g, 85%).

(±) 1-Aza-6α-H-4-thiabicyclo [4,4, 0]decane-7-one (D6) intermediate for compounds (3) and (4)

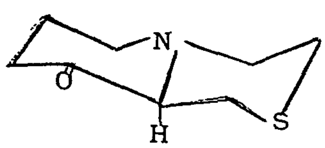

(D6)

Analogously, (±) ethyl 4-(3'ethoxycarbonyl) thiomorpholino butyrate (D5) (19 g) was converted into (±) 1-aza-6α-H-4-thiabicyclo[4,4,0]decane-&-one (D6) which was purified by column chromatography (silica, ether) (5.5 g, 48%)

(c) (±) 1-Aza-6α-H-4-oxabicyclo[4,4,0]decane-7-one (D3) intermediate for compounds (1) and (2)

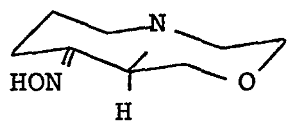

(D3)

An ethanolic (15 ml) solution of (±) 1-aza-6α-H-4-oxabicyclo[4,4,0]decane-7-one (D2) (1.5 g) was treated with pyridine (1.5 ml) and hydroxylamine hydrochloride (1.5 g) and the mixture was heated at reflux for 1 hour.. On cooling, the ethanol was evaporated and the residue was partitioned between potassium carbonate solution and methylene chloride (200 ml). Separation of the organic layer, drying ($K_2CO_3$) and concentration gave an oil, which, on trituration with ether, gave the (±) 1-aza-6α-H-4-oxabicyclo[4,4,0]decane-7-one oxime (D3) (1.3, 80%). mp 102-3°

Mass spectrum: ($M^+$)Found 170.1054, theory 170.1055.

(±) 1-Aza-6α-H-4-thiabicyclo[4,4,0]decane-7-one oxime (D7) intermediate for compounds (3) and (4)

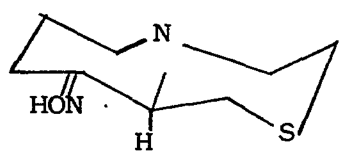

(D7)

Analogously, (±) 1-aza-6α-H-4-thiabicyclo [4,4,0]decane-7- one (D6) (5.5 g) was converted to (±) 1-aza-6α-H-4-thiabicyclo [4,4,0]decane-7-one oxime (D7 (3.5 g, 60%).

Mass Spectrum ($M^+$) Found 186.0819, theory 186.0827.

(d) <u>(±) 7α-Amino-1-aza-6α-H-4-oxabicyclo[4,4,0]decane (D4) intermediate for compounds (1) and (2)</u>

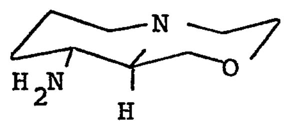

(D4)

Sodium (ca 1.1 g) was added, in portions over 20 minutes, to a refluxing solution of (±) 1-aza-6α-H-4-oxabicyclo[4,4,0]decane-7-one oxime (D3, 1.1 g) in amyl alcohol. When all the sodium had dissolved, the cooled reaction mixture was treated with 5N hydrochloric acid (ca 40 ml) and washed with ethyl acetate. The aqueous layer was saturated with potassium carbonate and re-extracted with methylene chloride (4 x 50 ml). The dried ($K_2CO_3$) methylene chloride solution was concentrated in vacuo to give the crude (±) 7α-amino-1-aza-6α-H-4-oxabicyclo[4,4,0]decane (D4) (0.8 g, 80%).

<u>(±) 7α,β-Amino-1-aza-6α-H-4-thiabicyclo[4,4,0] decane (D8) intermediate for compounds (3) and (4)</u>

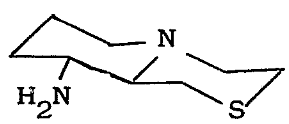

(D8)

To a stirred suspension of lithium aluminium hydride (1.4 g) in THF (100 ml) was added a solution of (±) 1-aza-6α-H-4-thiabicyclo[4,4,0]decane-7-one oxime (D7) (3.5 g) in THF (50 ml) and the mixture was heated at reflux for 12 hours. On cooling, water (1.4 ml), 2.5N sodium hydroxide (2.1 ml) and water (3 ml) was carefully added, and the reaction mixture was filtered. Evaporation and distillation gave the (±) 7α,β-amino-1-aza-6α-H-4-thiabicyclo[4,4,0]decane (D8) (2.4 g, 73%) bp 86-90°/0.1 mm.

Mass Spectrum, ($M^+$) Found 172.1039, theory 172.1034

(±) 7α,β-Amino-1-aza-6α-H-4-oxabicyclo[4,4,O]decane (D14) intermediates for compounds (11) and (12)

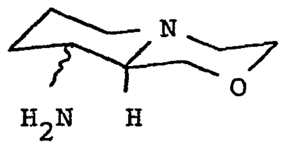

Analogously, (±) 1-aza-6-H-4-oxabicyclo[4,4,O]decane-7-one oxime (D.3) (2.1 g) was converted to the crude (±) 7α,β-amino-1-aza-6α-H-4-oxabicyclo[4,4,O] decane (D14) (2.1 g, 95%).

Description 2

(a) (±) Methyl (3-thiomorpholino)acetate (D9) intermediate for compounds 5, 6, 7, 8, 9 and 10

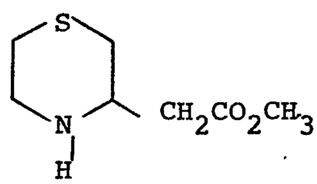

(D9)

To a stirred solution of methyl 4-bromocrotonate (7.2 ml) in chloroform (100 ml) at $0^{o}C$ was added, in one portion, a mixture of cysteamine hydrochloride (6.9 g) and triethylamine (20 ml) in chloroform (100 ml) and the whole was heated at reflux for 3 hours.

On cooling, the reaction mixture was neutralised with aqueous sodium bicarbonate solution and the organic layer was separated. Extraction of the aqueous layer with chloroform (2 x 50 ml) and evaporation of the combined chloroform extracts gave an oil, which was triturated with ether to remove the insoluble residue. Concentration of the decanted mother liquors gave the crude (±) methyl (3-thiomorpholine)acetate (D9) (12 g).

A pure sample was obtained by distillation, bp $100\text{-}4^{o}/0.5$ mm.

Mass Spectrum, $(MH^{+})$ 176 (L.I. $NH_3$).

N.m.r. ($\delta$, $CDCl_3$) 4.7-2.3 (m, all protons including 3.66, s, $OCH_3$).

(b) <u>(±) Methyl [3-(N-ethoxycarbonylmethyl)thiomorpholino]acetate (D10) intermediate for compounds 5, 6, 7, 8, 9 and 10.</u>

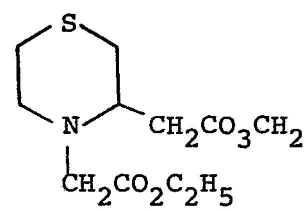

(D10)

A stirred suspension of potassium carbonate (6 g) in a solution of (±) methyl (3-thiomorpholino)acetate (D9) (6.5 g) and ethyl bromoacetate (4.3 ml) in acetone (100 ml) was heated at reflux for 2 days. On cooling, the acetone was evaporated in vacuo and the residue was extracted with ether. Concentration of the ether extract in vacuo and column chromatography of the oil (silica, ether) gave the (±) methyl [3-(N-ethoxycarbonylmethyl) thiomorpholino] acetate (D10) (8.6 g, 90%)

Mass Spectrum: ($M^+$) Found 261.1022, theory 261.1015.

N.m.r. (δ, $CDCl_3$) : 4.12 (q, 2H, $OC\underline{H}_2CH_3$);
3.64 (s, 3H, $OC\underline{H}_3$);
3.7-2.1 (m, 11H, remaining protons except)
1.25 (t, 3H, $OCH_2OC\underline{H}_3$).

(c) (±) 1-Aza-6α-H-4-thiabicyclo[4,3,0]nonane-8-one (D11) intermediate for compounds 5, 6, 7, 8, 9 and 10

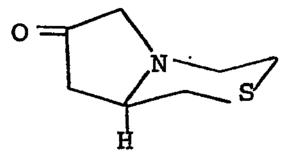

(D11)

Following the procedures detailed in Description 1(b), the (±) methyl [3-(N-ethoxycarbonylmethyl)thiomorpholino]acetate (D10) (8.6 g) was converted to (±) 1-aza-6α-H-4-thiabicyclo[4,3,0]nonane-8-one (D11) (4.05 g, 84%).

(d) (±) 1-Aza-6α-H-4-thiabicyclo[4,3,0]nonane-8-one oxime (D12) intermediate for compounds 5, 6, 7, 8, 9 and 10

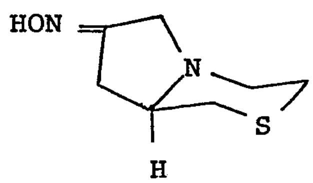

(D12)

Following the procedures detailed in Description 1(c), the (±) 1-aza-6α-H-4-thiabicyclo[4,3,0]nonane-8-one (D11) (4.0 g) was converted to (±) 1-aza-6α-H-4-thiabicyclo[4,3,0]nonane-8-one oxime (3.3 g, 62%).

M.p. 112-4$^{o}$ (ethyl acetate/petrol).

Mass Spectrum: ($M^{+}$) Found 172.0668, theory 172.0670.

(e) <u>(±) 8α,β-Amino-1-aza-6α-H-4-thiabicyclo[4,3,0]nonane (D13) intermediate for compounds 5, 6, 7, 8, 9 and 10</u>

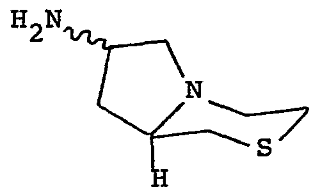

(D13)

Following the procedures detailed in Description 1(d), the (±) 1-aza-6α-H-4-thiabicyclo[4,3,0]nonane-8-one oxime (D12) (3.2 g) was converted to (±) 8α,β-amino-1-aza-6α-H-4-thiabicyclo[4,3,0]nonane (D13) (1.9 g. 62%) b.p. 86-8$^{o}$/0.1 mm.

Mass Spectrum, ($M^{+}$) 158.

Example 1

(±) 4-Acetamido-5-chloro-2-methoxy-N-(7α-1-aza-6α-H-4-oxabicyclo[4,4,0]decyl)benzamide (1)

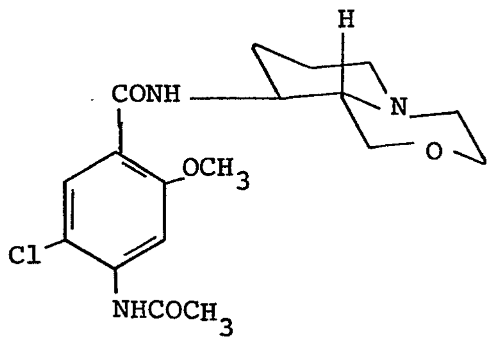

(1)

To a stirred solution of 4-acetamido-5-chloro-2-methoxy benzoic acid (1.6 g) in dry methylene chloride (100 ml) was added oxalyl chloride (0.58 ml) and dimethyl formamide (ca 5 drops) and the whole was stirred until gas evolution had ceased (ca 1 hour). The clear reaction mixture was cooled to $0^{o}C$ and triethylamine (2.5 ml) in methylene chloride (10 ml) was added followed by (±) 7α-amino-1-aza-6α-H-4-oxabicyclo[4,4,0]decane (D4) (0.8 g) in methylene chloride (10 ml). After stirring for 2 hours at room temperature, sodium hydroxide (2.5N, 12 ml) was added and the methylene chloride layer was separated, dried ($K_2CO_3$) and evaporated to give on purification by column chromatography (Alumina, 3% water, methylene chloride), the (±) 4-acetamido-5-chloro-2-methoxy-N-(7α-1-aza-6α-H-4-oxabicyclo[4,4,0] decyl)benzamide (1) (1.7 g, 89%).

N.m.r. (δ, $CDCl_3$) : 8.25 (s, 1H, aryl H);
8.10 (s, 1H, aryl H);
7.4 (br.s, 1H, $-NHCOCH_3$);
7.50 (br.d, 1H, CONHCH=);
4.4-1.0 (m, 20H, remaining protons including 3.92, s, 3H, $OCH_3$ and 2.24, s, 3H, $COCH_3$).

The following were prepared analogously:

**(±) 4-Acetamido-5-chloro-2-methoxy-N-(7β-1-aza-6α-H-4-thiabicyclo[4,4,0]decyl)benzamide (3) and (±) 4-Acetamido-5-chloro-2-methoxy-N-(7β-1-aza-6α-H-4-thiabicyclo[4,4,0]decyl)benzamide (13); from (D8)**

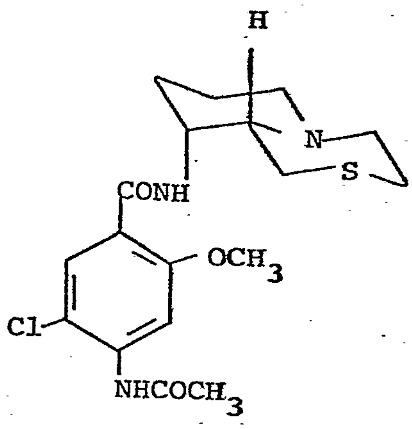

(3)

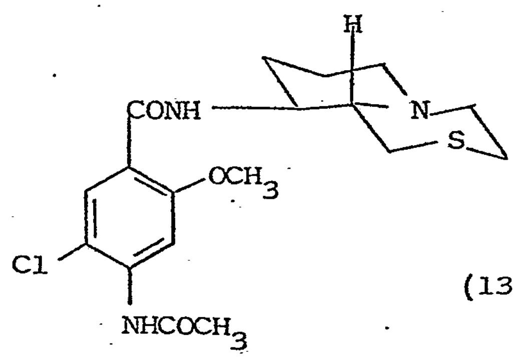

(13)

Chromatography (silica, ethyl acetate + 4% methanol) afforded the (±) 4-acetamido-5-chloro-2-methoxy-N-(7β-1-aza-6α-H-4-thiabicyclo[4,4,0]decyl)benzamide (3) (45%).

Mass spectrum, ($M^+$), Found 397.1223, theory 397.1227.

N.m.r. (δ, $CDCl_3$) : 8.36 (br.d, 1H, CON<u>H</u>CH=);
8.20 (s, 1H, aryl H);
8.06 (s, 1H, aryl H);
7.83 (br.s, 1H, NHCO$CH_3$);
4.25-3.7 (m, 4H, CONHCH=including 3.95, s, 3H, O$CH_3$);
3.25-1.2 (m, 16H, remaining protons including 2.25, s, 3H, CO$CH_3$).

Further elution afforded the (±) 4-acetamido-5-chloro-2-methoxy-N-(7α-1-aza-6α-H-4-thia bicyclo [4,4,0]decyl) benzamide (13) (40%)

N.m.r. (δ, $CDCL_3$) : 8.25 (s, IH, aryl H)
8.09 (s, IH, aryl H)
7.9-7.75 (br.s) IH, $NHCOCH_3$
7.6-7.35 (br.d, IH, NH CH=)
4.3-3.5 (m, 4H, CONHCH=
including 3.99,s, 3H, $OCH_3$
3.3-1.0 (m, 16H, remaining
protons including 2.25, s,
3H, $CDCL_3$)

<u>(±) 4-Acetamido-5-chloro-2-methoxy-N-(8-1-aza-6α-H-4-thiabicyclo[4,3,0]nonyl)benzamide less polar isomer</u> (5)

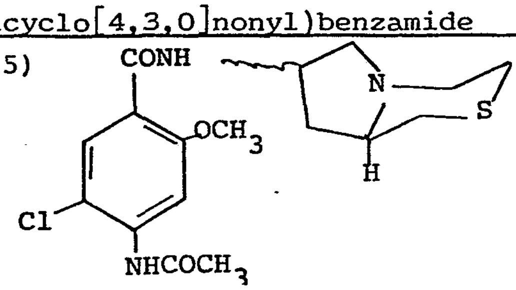

(5)

Chromatography (silica, ethyl acetate) afforded the less polar isomer (5) of (±) 4-acetamido-5-chloro-2-methoxy-N-(8-1-aza-6α-H-4-thiabicyclo[4,3,0]nonyl)benzamide (1.3 g, 26%) mp 176-8°.

Mass spectrum, ($MH^{\oplus}$) 384 (L.I. $NH_3$)

N.m.r. (δ, $CDCI_3$) : 8.33 (s, IH, aryl <u>H</u>);
8.20 (s, IH, aryl <u>H</u>);
8.3-7.7 (m, 2H, CON<u>H</u>CH= and $CH_3$CON<u>H</u>-);
4.8-4.2 (m, IH, CONHCH=);
3.88 (s, 3H, OC<u>H</u>$_3$);
3.5-2.0 (m, 13H, remaining protons including 2.2, s, 3H $CH_3$CO- except:)
1.6-1.1 (m, IH, aliphatic C-H)

as was the <u>more polar isomer (6)</u> (2.7 g ; 54%)

Mass Spectrum ($MH^+$) 384 (L.I. $NH_3$)

N.m.r. (δ, $CDCI_3$) 8.27 (s, IH, aryl <u>H</u>)
8.13 (s, IH, aryl <u>H</u>)
8.0-7.6 (M, 2H, CON<u>H</u>CH= and $CH_3$CON<u>H</u>-)
4.7-3.8 (m, 4H, CONHC<u>H</u>= and 3.7, s, 3H, OC<u>H</u>$_3$)
3.7-1.3 (m, 14H remaining protons including 2.26, s, 3H C<u>H</u>$_3$CO

($\pm$) 4-Acetamido-5-chloro-2-methoxy-N-(7β-1-aza-6α-H-4-oxabicyclo-[4,4,0]decyl)benzamide (11)

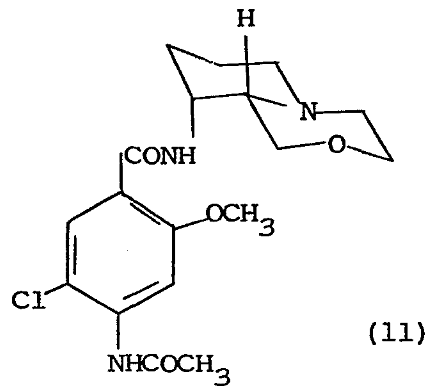

(II)

(11)

Analogously to the preparation of (3) was obtained a chromatographic first fraction of (I)4-acetamido-5-chloro-2-methoxy-N-(7β-1-aza-6α-H-4-oxabicyclo-[4,4,0]decyl)benzamide (11) (1.6 g, 35%) mp 203-5°.

Mass Spectrum ($M^+$) Found 381.1454. theory 381.1455

N.m.r. (δ, $CDCL_3$) : 8.6-8.3 (brd, IH, CONHCH=)

8.20 (s, IH, aryl H)

8.05 (s, IH, aryl H)

7.9-7.7 (brs, IH, $NHCOCH_3$

4.3-1.3 (m, 20H, remaining protons including 3.96, s, 3H, $OCH_3$ and 2.25, s, 3H, $COCH_3$).

Example 2

(±) 4-acetamido-5-chloro-2-methoxy-N-(8-1-aza-6α-H-4-oxothiabicyclo[4,3,0]nonyl)benzamide (9), more polar isomer

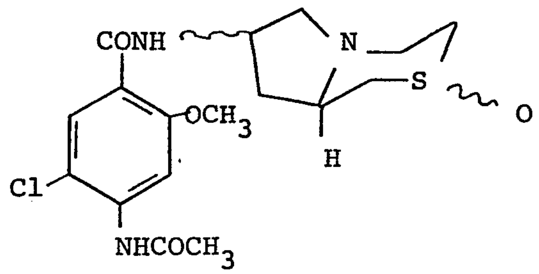

(9)

To a stirred solution of the more polar isomer of (±) 4-acetamido-5-chloro-2-methoxy-N-(8-1-aza-6α-H-4-oxothiabicyclo[4,3, 0]nonyl)benzamide (6) (0.7 g) in methanol (100 ml) was added in solution of sodium periodate (1.0g) in water (20 ml) and the whole was stirred, at room temperature, overnight. Removal of the solvent and extraction of the residue with hot chloroform gave the (±) 4-acetamido-5-chloro-2-methoxy-N-(8-1-aza-6α-H-4-oxothiabicyclo[4,3,0]nonyl)benzamide (9) (0.7 g), 95%) mp 185-9°.

Mass Spectrum ($M^+$) Found 399.1029, theory 399.1019

n.m.r. (δ, $CDCl_3$) 8.2-7.7 (m, 2H, CONHCH= and $CH_3CONH-$)
8.13 (s, 1H, aryl H)
7.96 (s, 1H, aryl H)
5.0-1.5 (m, 18H, remaining protons including 3.93, s, 3H, $-OCH_3$ and 2.27, s, 3H, $CH_3CONH-$)

The following were prepared analogously:

(±) 4-Acetamido-5-chloro-2-methoxy-N-(7α-1-aza-6α-H-4-oxothiabicyclo [4,4,0] decyl)benzamide (15), from (13)

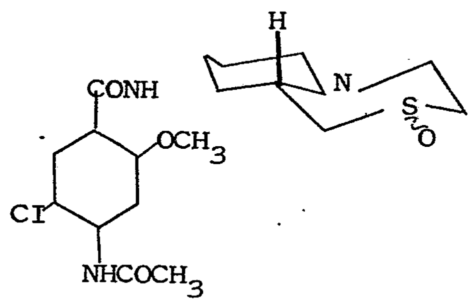

(15)

(85%) mp 234-6°.

Example 3

(±) 4-Amino-5-chloro-2-methoxy-N-(7α-1-aza-6α-H-4-oxabicyclo[4,4,0]decyl)benzamide hydrochloride (2)

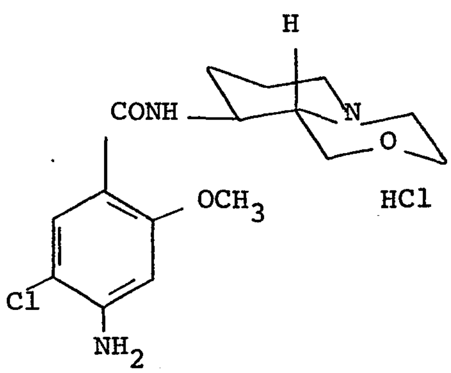

(2)

(1) (1.7 g) was heated at reflux with an aqueous ethanol (water 4 ml, ethanol 40 ml) solution of potassium hydroxide (1 g) for 2 hours. The mixture was then cooled to room temperature and the ethanol was removed by rotary evaporation. Extration with chloroform afforded the crude product as an oil. This was converted into its monohydrochloride by the addition of 1 mole of ethanolic hydrogen chloride and precipitation with ether to give the (±) 4-amino-5-chloro-2-methoxy-N-(7α-1-aza-6α-H-4-oxabicyclo[4,4,0]decyl)benzamide hydrochloride (2) (0.45 g, 20%) mp 245-7°.

N.m.r. (δ, $d^6$DMSO) : 7.85 (d,d, 1H, J=8.6Hz,0.4Hz, CONHCH=);
7.58 (s, 1H, H at $C_6$);
6.50 (s, 1H, H at $C_3$);
6.3-5.2 (m, 2H, $H_2N$);
4.5-1.3 (m, remaining protons including 3.82, s, 3H, $OCH_3$).

The following were prepared analogously:

(±) 4-Amino-5-chloro-2-methoxy-N-(7β-1-aza-6α-H-4-thiabicyclo[4,4,0]decyl)benzamide (4) from (3)

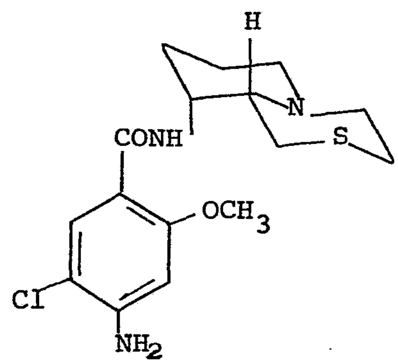

(4)

(80%) mp 170-1° (phase change 108-15°) ethyl acetate/petrol)

N.m.r. (δ, $CDCl_3$) : 8.32 (br.d, 1H, CONHCH=);
8.05 (s, 1H, H at $C_6$);
6.32 (s, 1H, H at $C_3$);
4.47 (br.s, 2H, $NH_2$);
4.18 (br.d, 1H, CONHCH=);
3.91 (s, 3H, $OCH_3$);
3.15-1.2 (m, 13H, remaining protons).

(±) 4-Amino-5-chloro-2-methoxy-N-(8-1-aza-6α-H-4-thia-bicyclo[4,3,0]nonyl-benzamide (7), less polar isomer, and (8), more polar isomer; from (5)

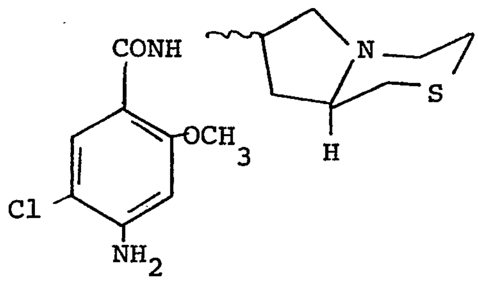

(7) and (8)

(7), less polar isomer (80%) mp 184-5° (ethyl acetate/petrol).

Mass Spectrum ($M^+$) 341

N.m.r. (δ, $CDCl_3$) 8.06 (s, 1H, H at $C_6$)
7.93-7.8 (m, 1H, CONHCH=)
6.27 (s, 1H, H at $C_3$)
4.6-4.3 (m, 2H, $NH_2$)
4.3-3.8 (m, 4H, CONHCH= including 3.86, s, 3H, $OCH_3$)
3.35-2.0 (m, 10H remaining protons except)
1.5-1.2 (m, 1H, aliphatic C-H)

(8) more polar isomer (60%). mp 196-7° (ethylacetate/petrol)

N.m.r. (δ, $CDCI_3$) 8.07 (s, IH, H at $C_6$)
7.78-7.64 (m, IH, CONHCH=)
6.29 (s, IH, H at $C_3$)
4.6-4.3 (m, 3H, $NH_2$ and CONH-CH=)
3.89 (s, 3H, $OCH_3$)
3.7-1.7 (m, IIH, remaining protons)

<u>(±) 4-amino-5-chloro-2-methoxy-N-(8-1-aza-6α-H-4-oxothiabicyclo[4,3,0]nonyl)benzamide (10), more polar isomer, from (9)</u>

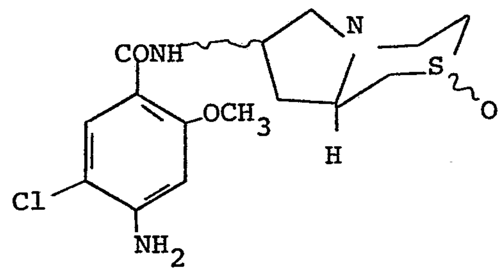

(10)

(60%) mp 191-3° (ethylacetate/petrol)

N.m.r. (δ, DMSO) 8.2-8.0 (m, 1H, CONHCH=)
7.91 (s, 1H, H at $C_6$)
6.48 (s, 1H, H at $C_3$)
5.4-5.0 (m, 2H, $NH_2$)
5.0-4.5 (m, 1H, CONHCH=)
4.3-1.7 (m, 14H, remaining protons including 3.93, s, 3H, -OCH3)

(±) 4-Amino-5-chloro-2-methoxy-N-(7β-1-aza-6a-H-4-oxabicyclo-[4,4,0]decyl)benzamide (12) from (11)

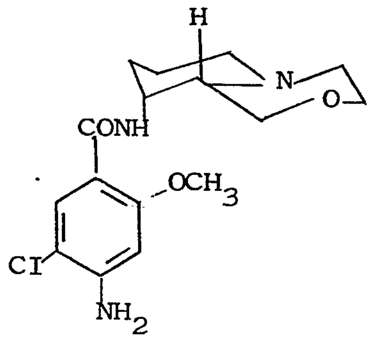

(12)

(70%) mp 104-6° (ethylacetate/petrol)

N.m.r. (δ, $CDCl_3$) 8.5-8.2 (br.d., IH, CONHCH=)
8.05 (s, IH, H at $C_6$)
6.32 (s, IH, H at $C_3$)
4.7-3.2 (m, 10H, aliphatic protons including 4.44, brs, 2H, $NH_2$ and 3.92, s, 3H, $OCH_3$)
3.0-0.9 (m, 9H, remaining protons)

(±) 4-Amino-5-chloro-2-methoxy-N-(7α-1-aza-6α-H-4-thiabicyclo [4,4,0]decyl)benzamide (14), from (13)

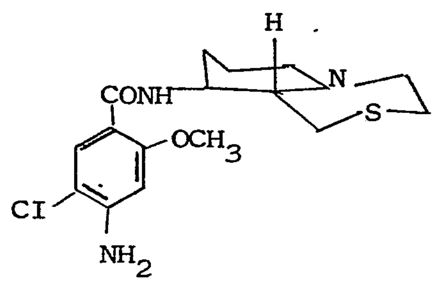

(14)

(70%) mp 178.80° (ethanol/water)

Mass Spectrum ($M^+$) Found 355.1044 Theory 355.1120

N.m.r. ($\delta$, $CDCI_3$)
8.06 (s, IH, H at $C_6$)
7.6-7.3 (brd, IH, NHCH=)
6.29 (s, IH, H at $C_3$)
4.42 (br.s, 2H, $NH_2$)
4.2-3.7 (m, 4H, CONH CH= including 3.88, s, 3H, $OCH_3$)
3.3-1.0 (m, 13H, remaining protons)

(±) 4-Amino-5-chloro-2-methoxy-N-(7α-1-aza-6α-H-4-oxothiabicyclo(4,4,0]decyl)benzamide (16) from (15)

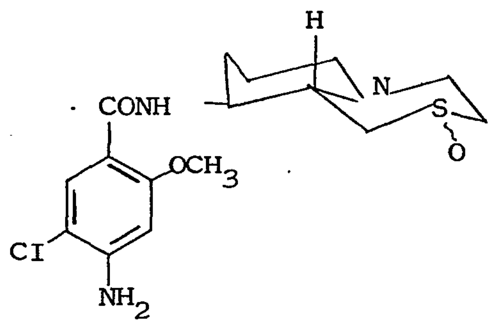

(16)

(50%) mp 224-6$^{o}$ (ethyl acetate)

Mass Spectrum ($M^+$) Found 371.1079, theory 371.1068

## Biological Data Section

### Gastric Activity

### Increase in intragastric pressure in the rat

Intragastric pressure changes were recorded from previously starved conscious but retrained rats using a saline filled catheter inserted into the lumen of the stomach via a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for the 40 minute period after the subcutaneous administration of the compounds. Students 't' test was applied to the difference in average values obtained for spontaneous and post-compound activity.

Compounds (2) and (14) were active in this test at 1.0 mg/kg subcutaneously.

### Toxicity

No toxic effects were observed in the test reported above.

CLAIMS

1. A compound of the formula (I):

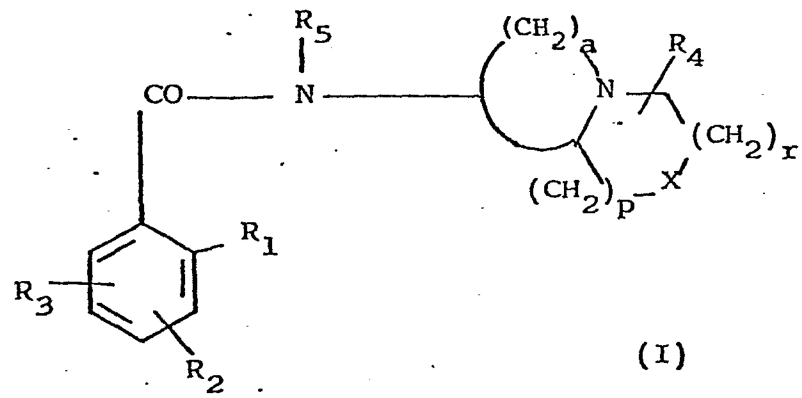

(I)

or a pharmaceutically acceptable salt thereof, wherein:

a is 3 to 5; and the $R_5$ substituted nitrogen atom is bound to one of the $-(CH_2)_a-$ carbon atoms, and has an ethan-1,2-diyl moiety linking it and the cyclic nitrogen atom;

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl-$S(O)_n$ wherein n is 0, 1 or 2, nitro, $C_{1-6}$ alkoxy, hydroxy, or amino, aminocarbonyl or aminosulphonyl optionally substituted by one or two $C_{1-6}$ alkyl groups;

or $R_1$ and $R_2$ taken together are methylenedioxy or ethylenedioxy in which case $R_3$ is any one of the groups given for $R_1$ and $R_2$ above;

$R_4$ is hydrogen, $C_{1-4}$ alkyl, phenyl or phenyl-$C_{1-4}$ alkyl;

$R_5$ is hydrogen or $C_{1-4}$ alkyl;

X is an oxygen or sulphur atom; or a sulphoxide group >S→O;

p is 1 or 2; and

r is 1 or 2.

2. A compound according to claim 1 of formula (VI):

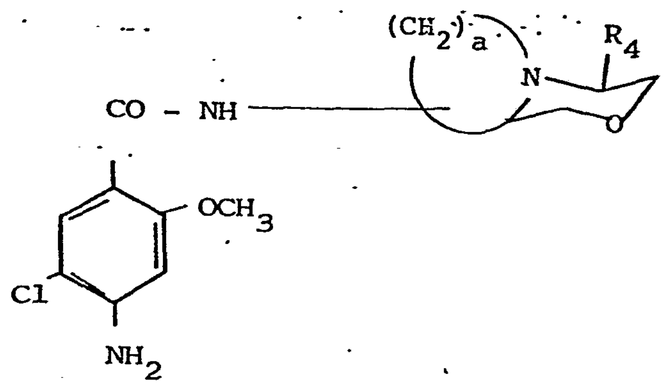

(VI)

3. A compound according to claim 1 of formula (VII):

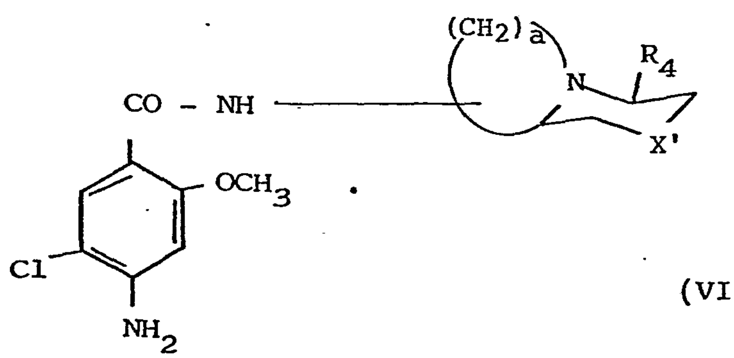

(VII)

wherein X' is S or SO.

4. A compound according to claim 3, wherein X' is S.

5. A compound according to claim 1 which is (+) 4-amino-5-chloro-2-methoxy-N-(7α-1-aza-6α-H-4-oxabicyclo[4,4,0]decyl)benzamide hydrochloride; (-) 4-amino-5-chloro-2-methoxy-N-(7α-1-aza-6α-H-4-oxabicyclo[4,4,0]decyl)benzamide hydrochloride; (+) 4-amino-5-chloro-2-methoxy-N-(7α-1-aza-6α-H-4-thiabicyclo[4,4,0]decyl)benzamide; or (-) 4-amino-5-chloro-2-methoxy-N-(7α-1-aza-6α-H-4-thiabicyclo[4,4,0]decyl)benzamide; or a pharmaceutically acceptable salt thereof.; or a mixture of the (+) and (-) enantiomers thereof.

6. A pharmaceutical composition comprising a compound according to claim 1 or a pharmaceutically acceptable salt thereof together with a pharmaceutically acceptable carrier.

7. A process for the preparation of a compound according to claim 1, which process comprises reacting an acid of the formula (VIII):

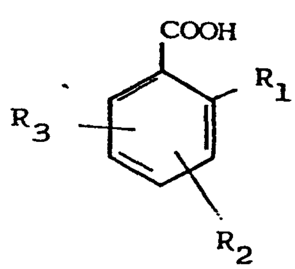

(VIII)

or a reactive derivative thereof, with a compound of the formula (IX):

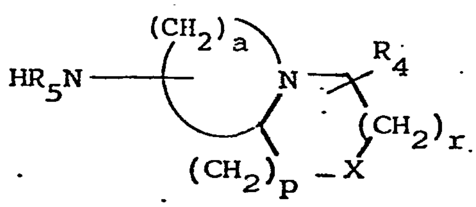

(IX)

wherein the variable groups are as defined in formula (I); and thereafter if desired or necessary converting a group $R_2$, $R_3$ or X in the thus formed compound to another group $R_2$, $R_3$ or X respectively.

8. A compound according to claim 1 or a pharmaceutically acceptable salt thereof for use in the treatment of disorders related to impaired gastrointestinal motility.

9. A method of treating disorders related to impaired gastrointestinal motility in mammals including humans comprising the administration of an effective amount of a compound according to claim 1 or a pharmaceutically acceptable salt thereof.

0057536

European Patent Office

# EUROPEAN SEARCH REPORT

Application number

EP 82 30 0323.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,A | EP - A2 - 0 034 015 (BEECHAM GROUP LTD.) * claims 1, 10, 11 * -- | 1,7,8 | C 07 D 513/04<br>C 07 D 498/04<br>A 61 K 31/535<br>A 61 K 31/54<br>//(C 07 D 513/04, 279/00,221/00)<br>(C 07 D 513/04, 279/00,209/00)<br>(C 07 D 498/04, 265/00,221/00) |
| D,A | DE - A1 - 2 748 260 (BEECHAM GROUP LTD.) & US - A - 4 213 983 -- | | |
| A | EP - A1 - 0 013 138 (BEECHAM GROUP LTD.) ---- | | |

TECHNICAL FIELDS SEARCHED (Int.Cl. 3)

A 61 K 31/535
A 61 K 31/54
C 07 D 498/04
C 07 D 513/04

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 14-04-1982 | FROELICH |

EPO Form 1503.1 06.78